(19) Europäisches Patentamt
European Patent Office
Office européen des brevets

(11) EP 0 764 022 B1

(12) # EUROPÄISCHE PATENTSCHRIFT

(45) Veröffentlichungstag und Bekanntmachung des Hinweises auf die Patenterteilung:
**20.12.2006 Patentblatt 2006/51**

(21) Anmeldenummer: **95922486.6**

(22) Anmeldetag: **26.05.1995**

(51) Int Cl.:
***A61K 31/00*** *(2006.01)* ***A61K 31/44*** *(2006.01)*
***A61K 31/53*** *(2006.01)* ***A61K 31/425*** *(2006.01)*
***A61K 31/505*** *(2006.01)* ***A61K 31/675*** *(2006.01)*
***A61K 31/415*** *(2006.01)* ***A61K 31/54*** *(2006.01)*
***A61P 33/10*** *(2006.01)*

(86) Internationale Anmeldenummer:
**PCT/EP1995/002014**

(87) Internationale Veröffentlichungsnummer:
**WO 1995/033453 (14.12.1995 Gazette 1995/53)**

(54) **AGONISTEN UND ANTAGONISTEN DER NICOTINERGEN ACETYLCHOLINREZEPTOREN VON INSEKTEN ALS ENDOPARASITIZIDE**

AGONISTS AND ANTAGONISTS OF THE NICOTINIC ACETYLCHOLINE RECEPTORS OF INSECTS TO CONTROL ENDOPARASITES

AGONISTES ET ANTAGONISTES DES RECEPTEURS NICOTINIQUES DE L'ACETYLCHOLINE DES INSECTES POUR LUTTER CONTRE DES ENDOPARASITES

(84) Benannte Vertragsstaaten:
**AT BE CH DE DK ES FR GB IE IT LI NL**

(30) Priorität: **07.06.1994 DE 4419814**

(43) Veröffentlichungstag der Anmeldung:
**26.03.1997 Patentblatt 1997/13**

(73) Patentinhaber: **Bayer HealthCare AG**
**51368 Leverkusen (DE)**

(72) Erfinder:
- **MENCKE, Norbert**
**D-51381 Leverkusen (DE)**
- **HARDER, Achim**
**D-51109 Köln (DE)**
- **HOPKINS, Terence**
**Tambourene, QLD (AU)**

(56) Entgegenhaltungen:
**EP-A- 0 154 178** **EP-A- 0 163 855**
**EP-A- 0 192 060** **EP-A- 0 259 738**
**EP-A- 0 398 084** **EP-A- 0 590 425**
**CA-A- 689 474**

- **'THE MERCK INDEX' 1989 , MERCK & CO. , RAHWAY, N.J. , U.S.A. "Nicotine" siehe Seite 1030**
- **SCIENCE, Bd. 261, 1993 Seiten 550-551, A.S. MOFFAT 'NEW CHEMICALS SEEK TO OUTWIT INSECT PESTS'**
- **NEUROPHARMACOLOGY, Bd. 27, Nr. 8, 1988 Seiten 843-848, R.D. PINNOCK ET AL. 'ACTIONS OF POTENT CHOLINERGIC ANTHELMINTICS (MORANTEL, PYRANTEL AND LEVAMISOLE) ON AN IDENTIFIED INSECT NEURONE REVEAL PHARMACOLOGICAL DIFFERENCES BETWEEN NEMATODE AND INSECT ACETYLCHOLINE RECEPTORS'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 764 022 B1

## Beschreibung

Die vorliegende Erfindung betrifft die Bekämpfung von Endoparasiten mittels Agonisten oder Antagonisten der nicotinergen Acetylcholinrezeptoren von Insekten.

Agonisten oder Antagonisten der nicotinergen Acetylcholinrezeptoren von Insekten sind bekannt. Zu ihnen gehören die Nicotinyl-Insektizide und ganz besonders die Chlornicotinyl-Insektizide. Es ist auch bekannt, daß diese Verbindungen hervorragend gegen pflanzenschädigende Insekten wirken. Die systemische Wirkung dieser Verbindungen in Pflanzen gegen pflanzenschädigende Insekten ist ebenfalls bekant.

Aus der PCT-Anmeldung WO 93/24 002 ist bekannt, daß sich bestimmte 1-[N-(Halo-3-pyridylmethyl)]-N-methylamino-1-alkylamino-2-nitroethylen-Derivate zur systemischen Anwendung gegen Flöhe bei Haustieren eignen. Bei dieser Art der Anwendung wird der Wirkstoff dem Haustier oral oder parenteral z.B. durch Injektion verabreicht und gelangt so in die Blutbahn des Haustieres. Die Flöhe nehmen den Wirkstoff dann beim Blutsaugen auf. Über eine Wirkung dieser Verbindungen gegen Endoparasiten ist jedoch nichts bekannt geworden.

Es wurde nun überraschenderweise gefunden, daß sich Agonisten oder Antagonisten der nicotinergen Acetylcholinrezeptoren von Insekten zur Bekämpfung von Endoparasiten eignen, nämlich von Haemonchus contortus.

Agonisten oder Antanogisten der nicotinogen Acetylcholinrezeptoren von Insekten sind bekannt z.B. aus Europäische Offenlegungsschriften Nr. 464 830, 428 941, 425 978, 386 565, 383 091, 375 907, 364 844, 315 826, 259 738, 254 859, 235 725, 212 600, 192 060, 163 855, 154 178, 136 636, 303 570, 302 833, 306 696, 189 972, 455 000, 135 956, 471 372, 302 389; Deutsche Offenlegungsschriften Nr. 3 639 877, 3 712 307; Japanische Offenlegungsschriften Nr. 03 220 176, 02 207 083, 63 307 857, 63 287 764, 03 246 283, 04 9371, 03 279 359, 03 255 072; US-Patentschriften Nr. 5 034 524, 4 948 798, 4 918 086, 5 039 686, 5 034 404; PCT-Anmeldungen Nr. WO 91/17 659, 91/4965; Französische Anmeldung Nr. 2 611 114; Brasilianische Anmeldung Nr. 88 03 621.

Auf die in diesen Publikationen beschriebenen Methoden, Verfahren, Formeln und Definitionen sowie auf die darin beschriebenen einzelnen Präparationen und Verbindungen wird hiermit ausdrücklich Bezug genommen.

Diese Verbindungen lassen sich durch die allgemeine Formel (I) wiedergeben

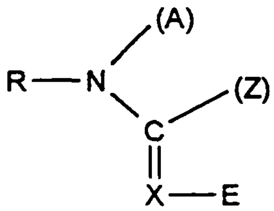

(I),

in welcher

R für Wasserstoff, gegebenenfalls substituierte Reste der Gruppe Acyl, Alkyl, Aryl, Aralkyl, Heteroaryl oder Heteroarylalkyl steht;

A für eine monofunktionelle Gruppe aus der Reihe Wasserstoff, Acyl, Alkyl, Aryl steht oder für eine bifunktionelle Gruppe steht, die mit dem Rest Z verknüpft ist;

E für einen elektronenziehenden Rest steht;

X für die Reste -CH= oder =N- steht, wobei der Rest -CH= anstelle eines H-Atoms mit dem Rest Z verknüpft sein kann;

Z für eine monofunktionelle Gruppe aus der Reihe Alkyl, -O-R, -S-R,

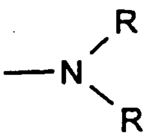

steht oder für eine bifunktionelle Gruppe steht, die mit dem Rest A oder dem Rest X verknüpft ist.

Besonders bevorzugt sind Verbindungen der Formel (I), in welcher die Reste folgende Bedeutung haben:

R steht für Wasserstoff sowie für gegebenenfalls substituierte Reste aus der Reihe Acyl, Alkyl, Aryl, Aralkyl, Heteroaryl, Heteroarylalkyl.

Als Acylreste seien genannt Formyl, Alkylcarbonyl, Arylcarbonyl, Alkylsulfonyl, Arylsulfonyl, (Alkyl-)-(Aryl-)-phosphoryl, die ihrerseits substituiert sein können.

Als Alkyl seien genannt $C_{1-10}$-Alkyl, insbesondere $C_{1-4}$-Alkyl, im einzelnen Methyl, Ethyl, i-Propyl, sec.- oder t.-Butyl, die ihrerseits substituiert sein können.

Als Aryl seien genannt Phenyl, Naphthyl, insbesondere Phenyl.

Als Aralkyl seien genannt Phenylmethyl, Phenethyl.

Als Heteroaryl seien genannt Heteroaryl mit bis zu 10 Ringatomen und N, O, S insbesondere N als Heteroatomen. Im einzelnen seien genannt Thienyl, Furyl, Thiazolyl, Imidazolyl, Pyridyl, Benzthiazolyl.

Als Heteroarylalkyl seien genannt Heteroarylmethyl, Heteroarylethyl mit bis zu 6 Ringatomen und N, O, S, insbesondere N als Heteroatomen.

Als Substituenten seien beispielhaft und vorzugsweise aufgeführt:

Alkyl mit vorzugsweise 1 bis 4, insbesondere 1 oder 2 Kohlenstoffatomen, wie Methyl, Ethyl, n- und i-Propyl und n-, i- und t-Butyl; Alkoxy mit vorzugsweise 1 bis 4, insbesondere 1 oder 2 Kohlenstoffatomen, wie Methoxy, Ethoxy, n- und i-Propyloxy und n-, i- und t-Butyloxy; Alkylthio mit vorzugsweise 1 bis 4, insbesondere 1 oder 2 Kohlenstoffatomen, wie Methylthio, Ethylthio, n- und i-Propylthio und n-, i- und t-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogen-atome gleich oder verschieden sind und als Halogenatome, vorzugsweise Fluor, Chlor oder Brom, insbesondere Fluor stehen, wie Trifluormethyl; Hydroxy; Halogen, vorzugsweise Fluor, Chlor, Brom und Jod, insbesondere Fluor, Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl-ethyl-amino, n- und i-Propylamino und Methyl-n-butylamino; Carboxyl; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl sowie Heteroarylamino und Heteroarylalkylamino wie Chlorpyridylamino und Chlorpyridylmethylamino.

A steht besonders bevorzugt für Wasserstoff sowie für gegebenenfalls substituierte Reste aus der Reihe Acyl, Alkyl, Aryl, die bevorzugt die bei R angegebenen Bedeutungen haben. A steht ferner für eine bifunktionelle Gruppe. Genannt sei gegebenenfalls substituiertes Alkylen mit 1-4, insbesondere 1-2 C-Atomen, wobei als Substituenten die weiter oben aufgezählten Substituenten genannt seien und wobei die Alkylengruppen durch Heteroatome aus der Reihe N, O, S unterbrochen sein können.

A und Z können gemeinsam mit den Atomen, an welche sie gebunden sind, einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei Alkyl der N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder. Als Beispiele für den heterocyclischen Ring seien Pyrrolidin, Piperidin, Piperazin, Hexamethylenimin, Hexahydro-1,3,5-triazin, Morpholin genannt, die gegebenenfalls bevorzugt durch Methyl substituiert sein können.

E steht für einen elektronenziehenden Rest, wobei insbesondere $NO_2$, CN, Halogenalkylcarbonyl wie 1,5-Halogen-$C_{1-4}$-carbonyl, insbesondere $COCF_3$ genannt seien.

X steht für -CH= oder -N=

Z steht für gegebenenfalls substituierte Reste Alkyl, -OR, -SR, -NRR, wobei R und die Substituenten bevorzugt die oben angegebene Bedeutung haben.

Z kann außer dem obengenannten Ring gemeinsam mit dem Atom, an welches es gebunden ist und dem Rest

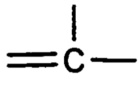

an der Stelle von X einen gesättigten oder ungesättigten heterocyclischen Ring bilden. Der heterocyclische Ring kann weitere 1 oder 2 gleiche oder verschiedene Heteroatome und/oder Heterogruppen enthalten. Als Heteroatome stehen vorzugsweise Sauerstoff, Schwefel oder Stickstoff und als Heterogruppen N-Alkyl, wobei die Alkyl oder N-Alkyl-Gruppe vorzugsweise 1 bis 4, insbesondere 1 oder 2 Kohlenstoffatome enthält. Als Alkyl seien Methyl, Ethyl, n- und i-Propyl und n-, i- und t-Butyl genannt. Der heterocyclische Ring enthält 5 bis 7, vorzugsweise 5 oder 6 Ringglieder.

Als Beispiele für den heterocyclischen Ring seien Pyrrolidin, Piperidin, Piperazin, Hexamethylenimin, Morpholin und N-Methylpiperazin genannt.

Als ganz besonders bevorzugt erfindungsgemäß verwendbare Verbindungen seien Verbindungen der allgemeinen Formeln (II) und (III) genannt:

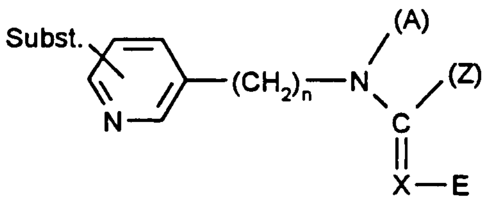

(II),

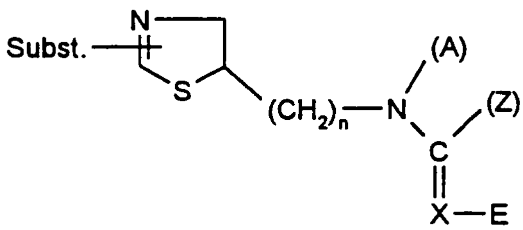

(III),

in welchen

n für 1 oder 2 steht,

Subst. für einen der oben aufgeführten Substituenten, insbesonders für Halogen, ganz besondere für Chlor, steht,

A, Z, X und E die oben angegebenen Bedeutungen haben.

Im einzelnen seien folgende Verbindungen genannt:

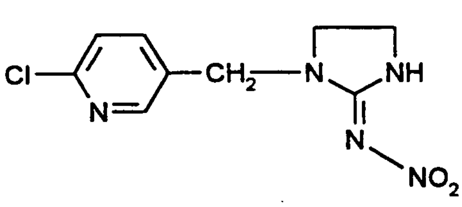

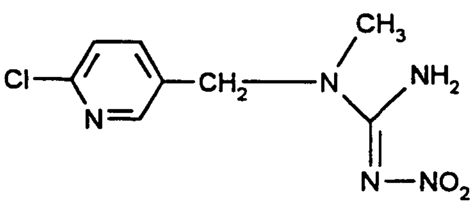

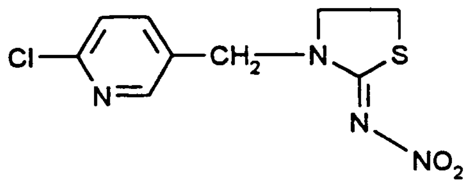

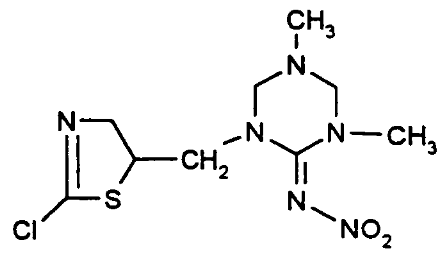

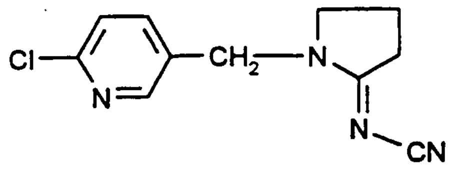

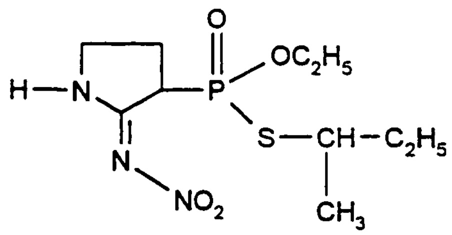

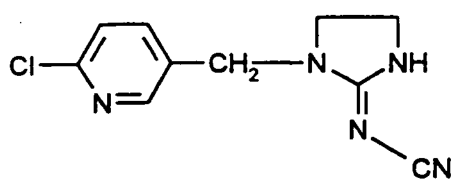

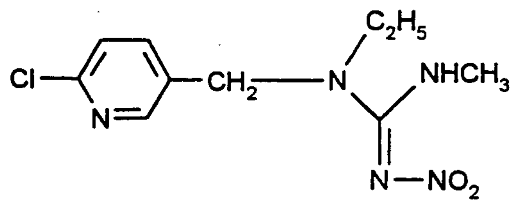

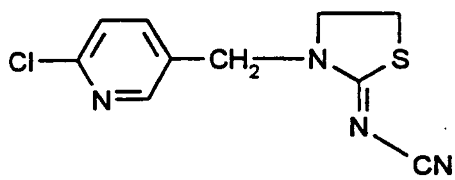

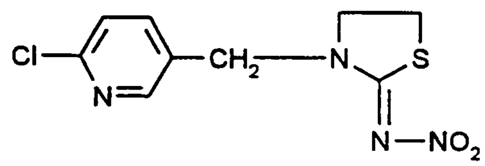

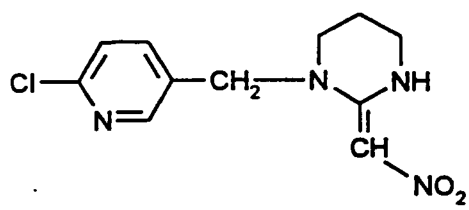

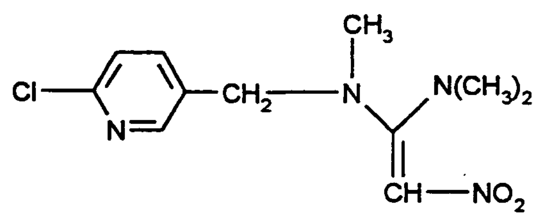

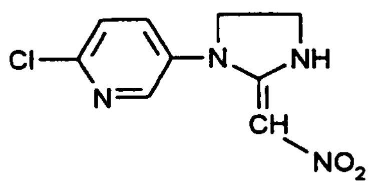

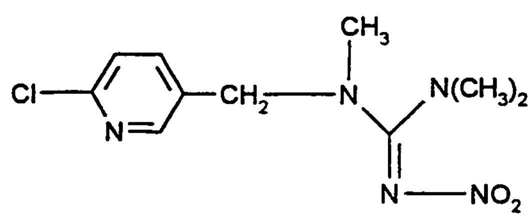

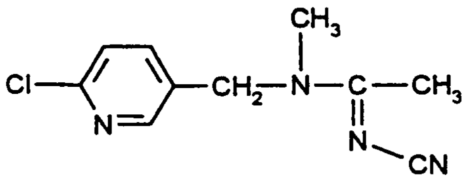

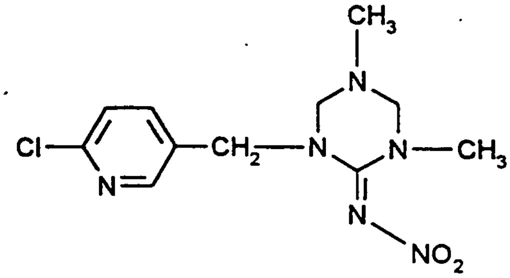

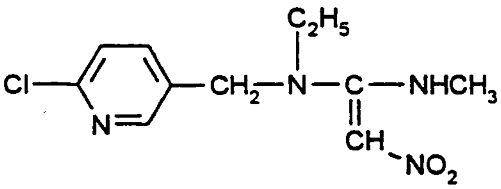

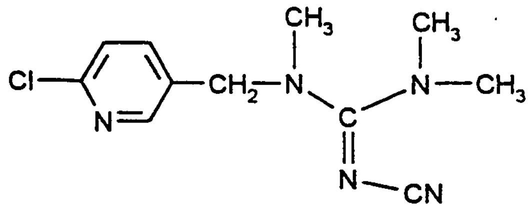

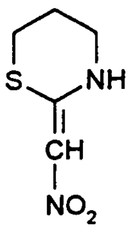

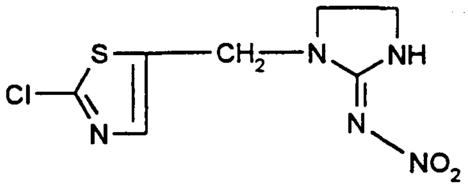

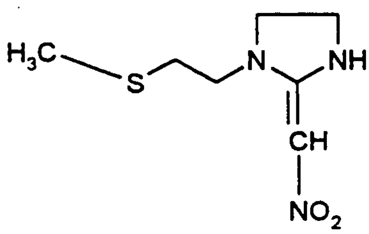

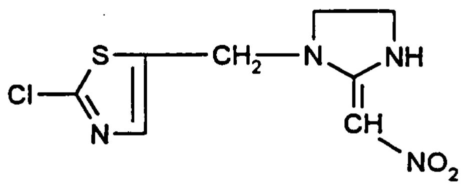

Die Wirkstoffe eignen sich bei günstiger Warmblütertoxizität zur Bekämpfung von pathogenen Endoparasiten, die bei Menschen und in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Arten wirksam. Durch die Bekämpfung der pathogenen Endoparasiten sollen Krankheit, Todesfälle und Leistungsminderungen (z.B. bei der Produktion von Fleisch, Milch, Wolle, Häuten, Eiern, Honig usw.) sowie gegebenenfalls auch Übertragung auf den Menschen vermindert werden, so daß durch den Einsatz der Wirkstoffe eine wirtschaftlichere und einfachere Tierhaltung möglich ist. Zu den pathogenen Endoparasiten zählen Nematoden nämlich Haemonchus contortus.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten.

Zu Labor- und Versuchstieren gehören Mäuse, Ratten, Meerschweinchen, Goldhamster, Hunde und Katzen.

Zu den Hobbytieren gehören Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral,

dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markierungsvorrichtungen.

Die enterale Anwendung der Wirkstoffe geschieht z.B. oral in Form von Pulver, Zäpfchen, Tabletten, Kapseln, Fasten, Tränken, Granulaten, Drenchen, Boli, medikiertem Futter oder Trinkwasser. Die dermale Anwendung geschieht z.B. in Form des Tauchens (Dippen), Sprühens (Sprayen), Badens, Waschens, Aufgießens (pour-on and spot-on) und des Einpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspensionen zur oralen oder dermalen Anwendung sowie zu Injektion; Halbfeste Zubereitungen;

Formulierungen bei denen der Wirkstoff in einer Salbengrundlage oder in einer Öl in Wasser oder Wasser in Öl Emulsionsgrundlage verarbeitet ist;

Feste Zubereitungen wie Pulver, Premixe oder Konzentrate, Granulate, Pellets, Tabletten, Boli, Kapseln; Aerosole und Inhalate, wirkstofflialtige Formkörper.

Injektionslösungen werden intravenös, intramusculär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zu Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden wie oben bei den Injektionslösungen beschrieben hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen, Baden oder Waschen aufgebracht. Diese Lösungen werden wie oben bei den Injektionslösungen beschrieben hergestellt.

Es kann vorteilhaft sein, bei der Herstellung Verdickungsmittel zuzufügen. Verdickungsmittel sind: Anorganische Verdickungsmittel wie Bentonite, kolloidale Kieselsäure, Aluminiummonostearat, organische Verdickugsmittel wie Cellulosederivate, Polyvinylalkohole und deren Copolymere, Acrylate und Metacrylate.

Gele werden auf die Haut aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgieß-Formulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgieß-Formulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglykole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglycolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfat, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektion angewendet werden.

Emulsionen sind entweder vom Typ Wasser in Öl oder vom Typ Öl in Wasser.

Sie werden hergestellt, indem man den Wirkstoff entweder in der hydrophoben oder in der hydrophilen Phase gelöst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäure der Kettenlänge $C_{8-12}$ oder andren speziell ausgewählten natürlichen Fettsäuren, Partialglyceridgemische gesättigter oder ungesättigter eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Cypryl/Caprinsäureester von gesättigten Fettalkoholen der Kettelänge $C_{12}$-$C_{18}$, Isopropylstearat, Ölsäureoleylester, Ölsäuredecylester, Ethyloleat, Milchsäureethylester, wachsartige Fettsäureester wei künstliches Entenbürzeldrüsenfett, Dibutylphthalat, Adipinsäurediisopropylester, letzterem verwandte Estergemische u.a.

Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:

Wasser, Alkohole wie z.B. Propylenglykol, Glycerin, Sorbitol und ihre Gemische.

Als Emulgatoren seien genannt: nichtionogene Tenside, z.B. polyoxyethyliertes Rizinusöl, polyoxyethyliertes Sorbitan-monooleat, Sorbitanmonostearat, Glycerinmonostearat, Polyoxyethylstearat, Alkylphenolpolyglykolether;
ampholytische Tenside wie Di-Na-N-lauryl-β-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchlorid.

Als weitere Hilfsstoffe seien genannt: Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebene Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker-, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen, die gegen pathogene Endoparasiten wirken, vorliegen. Solche Wirkstoffe sind z.B. L-2,3,5,6-Tetrahydro-6-phenyl-imidazolthiazol, Benzimidazolcarbamate, Praziquantel, Pyrantel, Febantel.

Anwendungsfertige Zubereitungen enthalten den Wirkstoff in Konzentrationen von 10 ppm bis 20 Gew.-%, bevorzugt von 0,1 bis 10 Gew.-%.

Zubereitungen die vor Anwendung verdünnt werden, enthalten den Wirkstoff in Konzentrationen von 0,5 bis 90 Gew.-%, bevorzugt von 5 bis 50 Gew.-%.

Im allgemeinen hat es sich als vorteilhaft erwiesen, Mengen von etwa 1 bis 100 mg Wirkstoff je kg Körpergewicht pro Tag zur Erzielung wirksamer Ergebnisse zu verabreichen.

Als Wirkstoff wird in den folgenden Beispielen Imidachloprid = 1-[(6-Choro-3-pyridinyl)methyl]-N-nitro-imidazolidinimine eingesetzt.

**Beispiel 1**

SC-(Suspensionskonzentrat) Formulierung:

368 g Imidacloprid
35 g Blockpolymer aus Emulgator
Ethylenoxid- und Propylenoxid
12 g Ditolylethersulfonat-Formaldehyd-Kondensat (Emulgator)
3,5 g wasserlöslicher Polyvinylalkohol
58,0 g $NH_4Cl$
116,0 g Harnstoff
1,2 g (37 %ige wäßrige Salzsäure)
4,6 g Xanthan-gum
560,5 g destilliertes Wasser

**Beispiel 2**

WP (dispergierbares Pulver)-Formulierung:

25,0 g Imidacloprid
1,0 g Diisobutyl-naphthalin-Na-sulfonat
10,0 g n-Dodecylbenzylsulfonsäure Calcium
12,0 g hochdisperser Kieselsäure-haltiger Alkylarylpolyglykolether
3,0 g Ditolylethersulfonat-Formaldehyd-Kondensat (Emulgator)
2,0 g ®Baysilon-E, ein siliconhaltiger Entschäumer der Fa. Bayer AG
2,0 g feindisperses Siliciumdioxid und
45,0 g Kaolin

**Beispiel 3**

SL-(wasserlösliche Konzentrat)-Formulierung

18,3 g Imidacloprid
2,5 g neutraler Emulgator auf Basis Alkylarylpolyglykolether
3,5 g Natriumsulfobernsteinsäuredüsooctylester
38,4 g Dimethylsulfoxid und
37,5 g 2-Propanol

**Beispiel 4**

SL-(wasserlösliche Konzentrat)-Formulierung

185, g Imidacloprid
5,0 g Natriumsulfonberasteinsäuredüsooctylester und
76,5 g Dimethylsulfoxid

werden einer 100 g Schampoo-Formulierung bestehend aus

| | |
|---|---|
| 44,4 Gew.-% | Marlon AT 50, ein Triethanolaminsalz von Alkylbenzolsulfonsäuren der Fa. Hüls AG |
| 11,1 Gew.-% | Marlon A 350, Natriumsalz von Alkylbenzolsulfonsäuren der Fa. Hüls AG |
| 3,0 Gew.-% | Kondensationsprodukt von Ölsäuren und Diethanolamin der Fa. Hüls AG und |
| 41,5 Gew.-% | Polyethylenglykol |

beigemengt.

**Beispiel 5**

Spray-Formulierung bestehend aus

2,0 g Imidacloprid
10,0 g Dimethylsulfoxid
35,0 g 2-Propanol und
53,0 g Aceton

**Beispiel A**

In vivo Nematodentest

Haemonchus contortus / Schaf

Experimentell mit Haemonchus contortus infizierte Schafe wurden nach Ablauf der Präpatenzzeit des Parasiten behandelt. Die Wirkstoffe wurden als reiner Wirkstoff in Gelatinekapseln oral appliziert.

Der Wirkungsgrad wird dadurch bestimmt, daß man die mit dem Kot ausgeschiedenen Wurmeier vor und nach der Behandldung quantitativ auszählt.

Ein völliges Sisterieren der Eiausscheidung nach der Behandlung bedeutet, daß die Würmer abgetrieben wurden oder so geschädigt sind, daß sie keine Eier mehr produzieren (Dosis effectiva).

Geprüfte Wirkstoffe und wirksame Dosierungen (Dosis effectiva) sind aus der nachfolgenden Tabelle ersichtlich.

| Wirkstoff | Dosis effectiva in mg/kg |
|---|---|
| Imidacloprid | 10 |

**Referenz - Beispiel B**

Beispiel Hymenolepis nana / Maus

Experimentell mit infektiösen Eiern aus Proglottiden oral infiziert. Nach Ablauf der Präpatenzzeit wird behandelt (4 mal an 4 aufeinander folgenden Tagen oral). Nach 7 Tagen wird im Darm die Anzahl der Scolices ermittelt. Die Wirksamkeit wird berechnet nach der Formel

$$\%\ \text{Wirksamkeit} = \frac{\text{Anzahl Scolices Kontrollgruppe} - \text{Anzahl Scolices der Behandlungsgruppe}}{\text{Anzahl Scolices in Kontrollgruppe}}$$

Wirkstoff: Imidacloprid; Wirksamkeit: 100 % bei oraler Applikation von 25 mg/kg.

## Patentansprüche

**1.** Verwendung von Agonisten oder Antagonisten der nicotinergen Acetylcholinrezeptoren von Insekten der Formel (I)

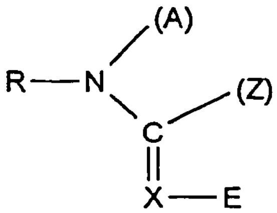

(I),

in welcher

R für Wasserstoff, gegebenenfalls substituierte Reste der Gruppe Acyl, Alkyl, Aryl, Aralkyl, Heteroaryl oder Heteroarylalkyl steht;
A für eine monofunktionelle Gruppe aus der Reihe Wasserstoff, Acyl, Alkyl, Aryl steht oder für eine bifunktionelle Gruppe steht, die mit dem Rest Z verknüpft ist;
E für einen elektronenziehenden Rest steht;
X für die Reste -CH= oder =N- steht, wobei der Rest -CH= anstelle eines H-Atoms mit dem Rest Z verknüpft sein kann;
Z für eine monofunktionelle Gruppe aus der Reihe Alkyl, -O-R, -S-R,

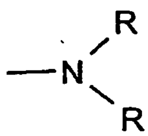

steht
oder für eine bifunktionelle Gruppe steht, die mit dem Rest A oder dem Rest X verknüpft ist,

zur Herstellung von Arzneimitteln zur Bekämpfung von Haemonchus contortus bei Menschen oder Tieren

**2.** Verwendung gemäß Anspruch 1 von Verbindungen der Formeln (II) oder (III)

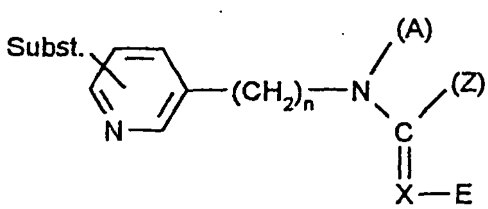

(II),

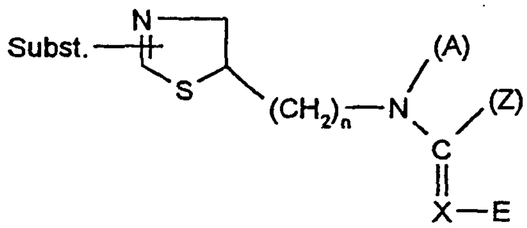

(III),

in welchen

n für 1 oder 2 steht,
Subst. für einen der oben aufgeführten Substituenten, insbesonders für Halogen, ganz besondere für Chlor, steht,
A, Z, X, und E die in Anspruch 1 angegebenen Bedeutung haben.

**3.** Verwendung gemäß Anspruch 1 der Verbindungen

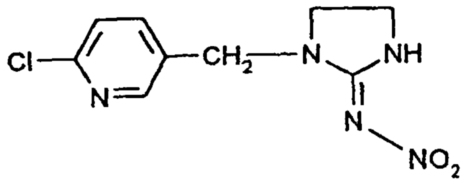

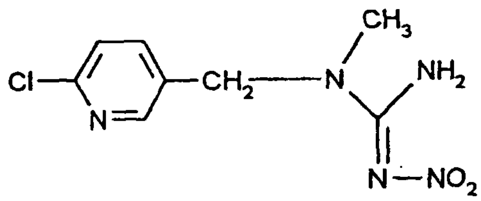

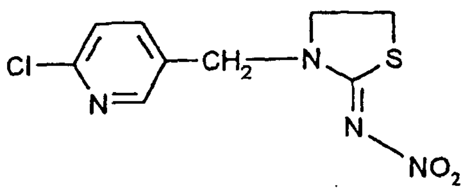

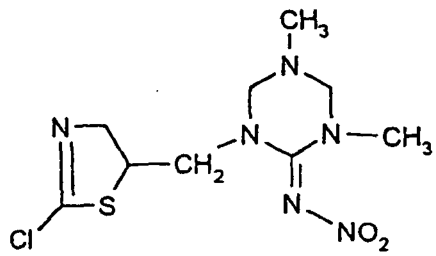

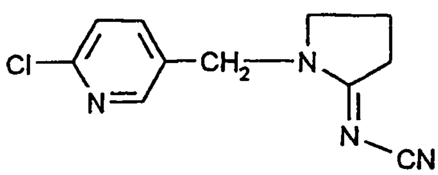

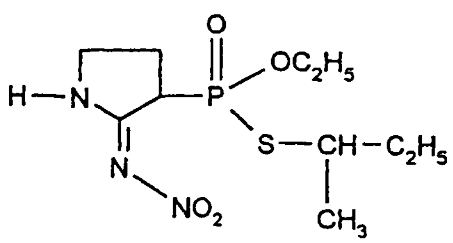

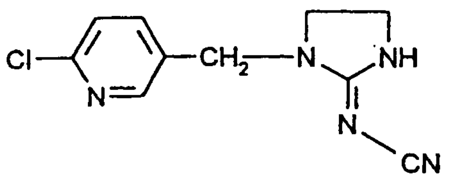

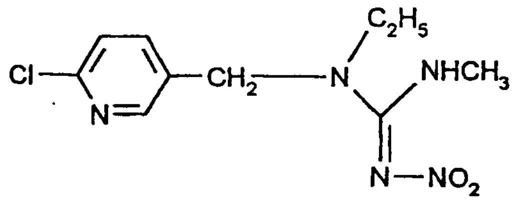

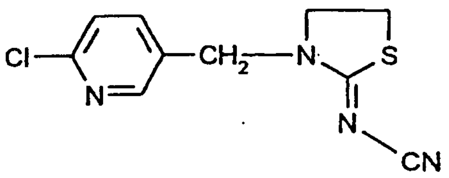

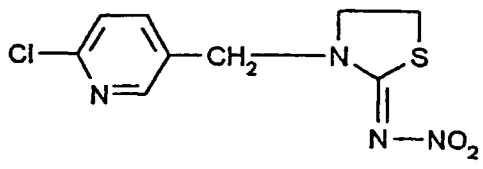

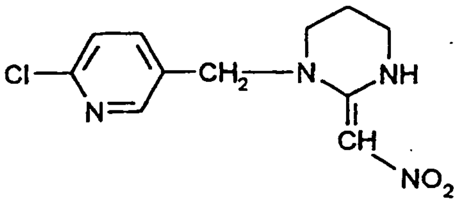

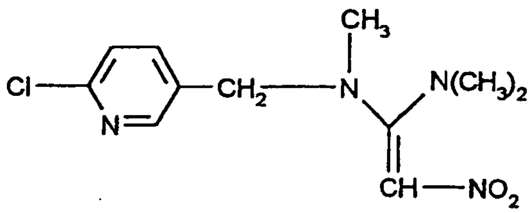

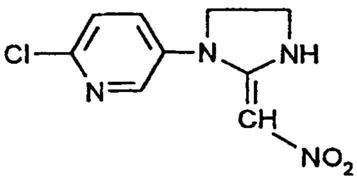

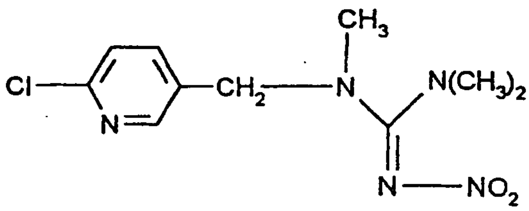

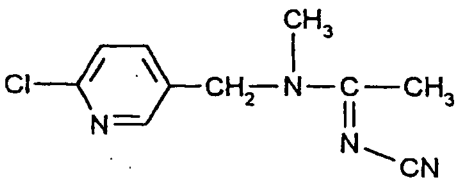

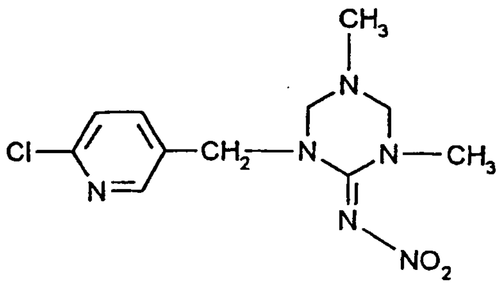

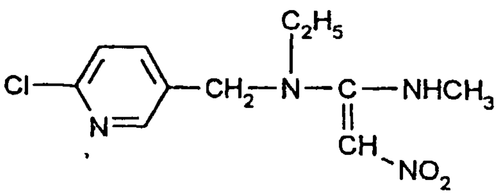

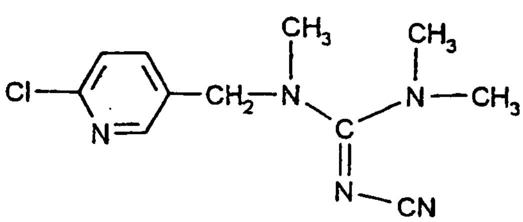

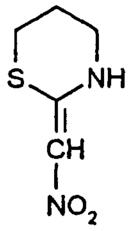

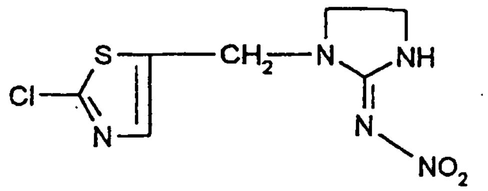

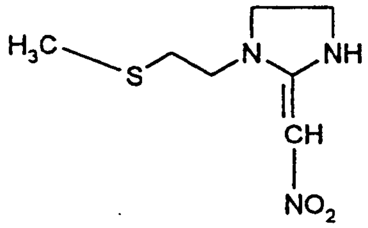

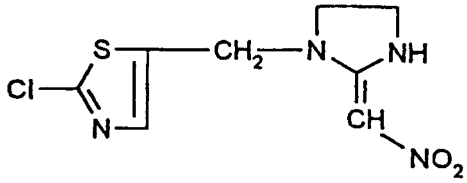

## Claims

1. Use of agonists or antagonists of the nicotinergic acetylcholine receptors of insects of the formula (I)

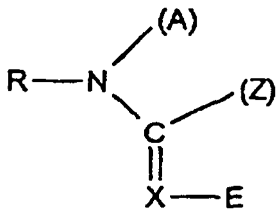

(I),

in which

R represents hydrogen, optionally substituted radicals from the group consisting of acyl, alkyl, aryl, aralkyl, heteroaryl or heteroarylalkyl;
A represents a monofunctional group from the series hydrogen, acyl, alkyl and aryl or represents a bifunctional group which is linked to the radical Z;
E represents an electron-withdrawing radical;
X represents the radicals -CH= or =N-, it being possible for the radical -CH= to be linked to the radical Z instead of an H atom;
Z represents a monofunctional group in the series consisting of alkyl, -O-R, -S-R

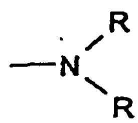

or represents a bifunctional group which is linked to the radical A or the radical X

for the preparation of medicaments for controlling Haemonchus contortus in humans or animals.

**2.** Use according to Claim 1 of compounds of the formula (II) or (III)

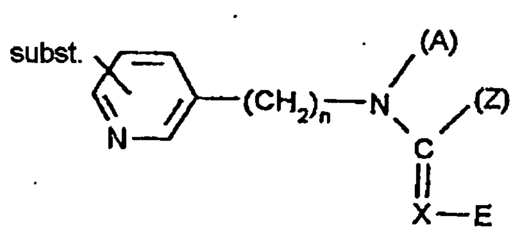

(II),

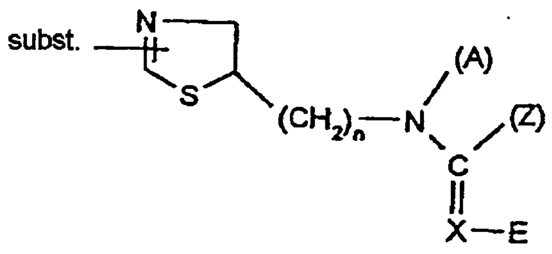

(III),

in which

n represents 1 or 2,
subst. represents one of the abovementioned substituents, in particular halogen, very especially chlorine,
A, Z, X and E have the meanings given in Claim 1.

**3.** Use according to Claim 1 of the compounds

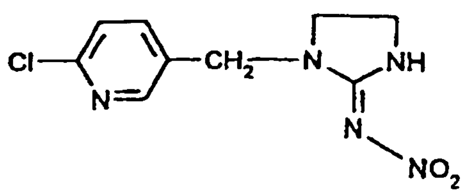

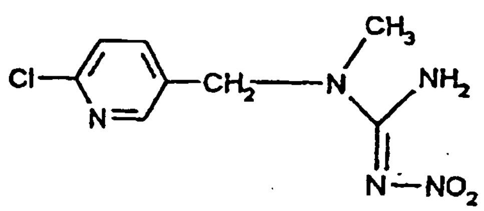

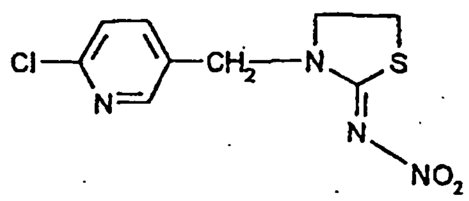

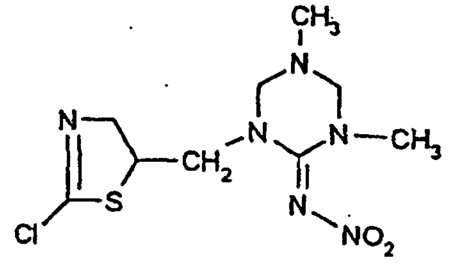

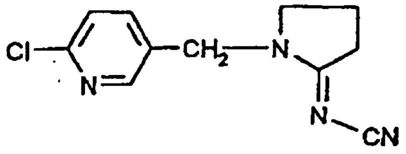

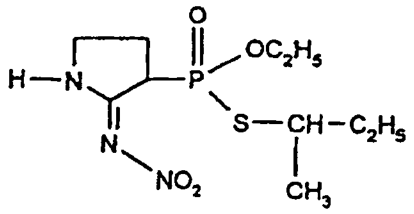

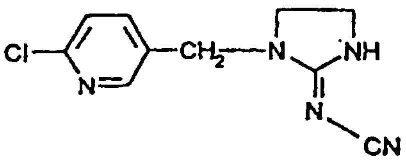

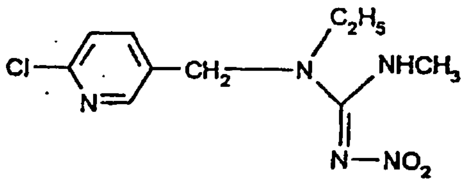

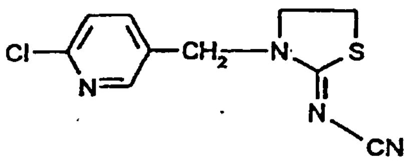

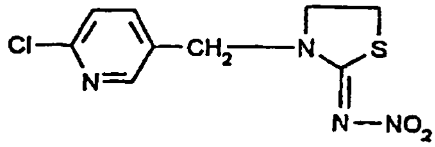

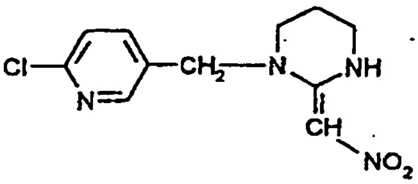

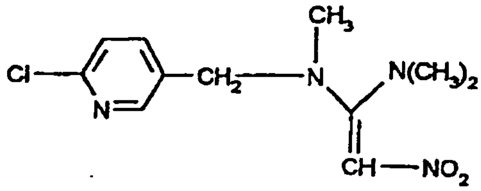

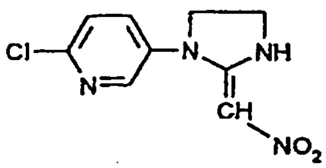

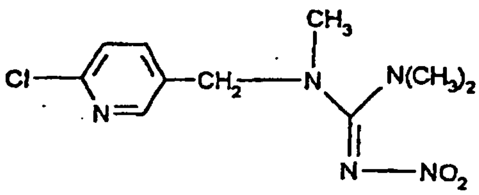

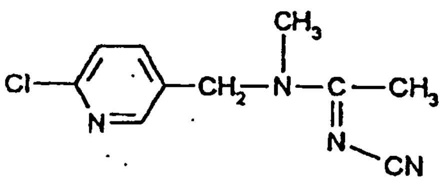

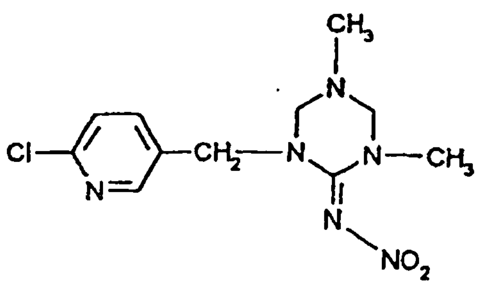

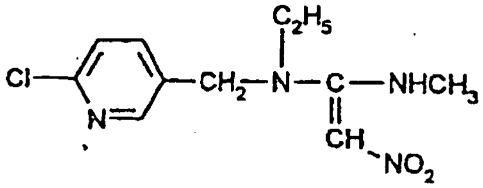

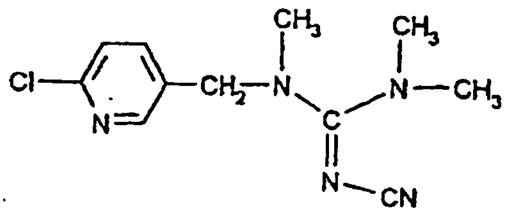

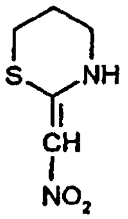

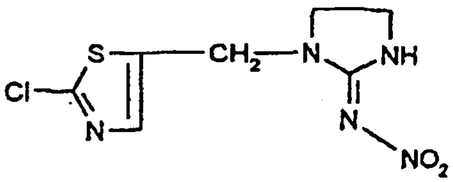

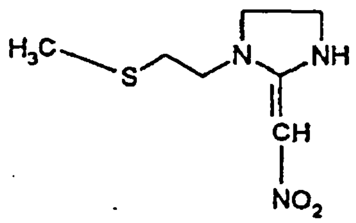

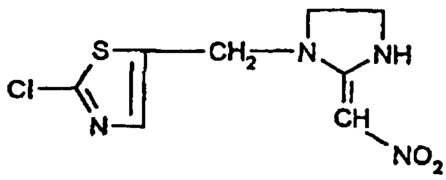

## Revendications

1. Utilisation d'agonistes ou d'antagonistes des récepteurs nicotinergiques d'acétylcholine d'insectes, de formule (I)

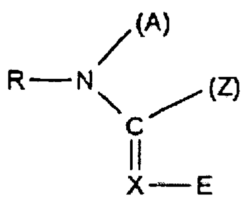

(I),

dans laquelle

R représente l'hydrogène, des restes éventuellement substitués du groupe des restes acyle, alkyle, aryle, aralkyle, hétéroaryle ou hétéroarylalkyle ;
A représente un groupe monofonctionnel de la série hydrogène, acyle, alkyle, aryle ou un groupe bifonctionnel qui est lié au reste Z ;
E représente un reste électroattractif ;
X désigne les restes -CH= ou =N-, le reste -CH= pouvant être lié au reste Z à la place d'un atome H ;
Z désigne un groupe monofonctionnel de la série alkyle, -O-R, -S-R,

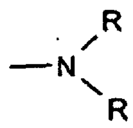

ou un groupe bifonctionnel qui est lié au reste A ou au reste X,

pour la préparation de médicaments destinés à combattre Haemonchus contortus en médecine humaine ou en médecine vétérinaire.

**2.** Utilisation suivant la revendication 1 de composés de formules (II) ou (III)

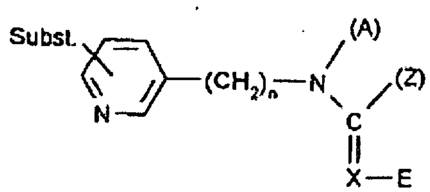

(II),

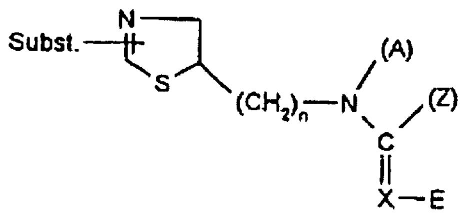

(III),

dans lesquelles

n a la valeur 1 ou 2
Subst. désigne l'un des substituants indiqués ci-dessus, en particulier un halogène, notamment le chlore,
A, Z, X et E ont la définition indiquée dans la revendication 1.

**3.** Utilisation suivant la revendication 1 des composés